# EUROPEAN PATENT SPECIFICATION

(11) **EP 3 642 746 B1**
(45) Date of publication and mention of the grant of the patent: **01.11.2023**
(21) Application number: 18731102.2
(22) Date of filing: 19.06.2018
(51) Int. Cl.: G16B 30/00, G16B 45/00

(54) **METHOD AND APPARATUS FOR PERFORMING A NORMALIZATION IN THE CONTEXT OF SEQUENCING ANALYSIS**
VERFAHREN UND VORRICHTUNG ZUR DURCHFÜHRUNG EINER NORMALISIERUNG IM KONTEXT EINER SEQUENZIERUNGSANALYSE
PROCÉDÉ ET APPAREIL PERMETTANT DE RÉALISER UNE NORMALISATION DANS LE CONTEXTE D'UNE ANALYSE DE SÉQUENÇAGE

(30) Priority: 20.06.2017 EP 17177001
(43) Date of publication of application: 29.04.2020
(73) Proprietor: Qiagen GmbH, 40724 Hilden (DE)
(72) Inventor: OESTERREICH CARRILLO, Fernando, 40724 Hilden (DE); LOHEL, Maiko, 40724 Hilden (DE); ZERFASS, Thorsten, 40724 Hilden (DE)
(74) Representative: Pfitzner, Hannes
(86) International application number: PCT/EP2018/066278
(87) International publication number: WO 2018/234314

(56) References cited:
- C. YE ET AL: "BlindCall: ultra-fast base-calling of high-throughput sequencing data by blind deconvolution", BIOINFORMATICS., vol. 30, no. 9, 9 January 2014 (2014-01-09), pages 1214-1219, XP055390417, GB ISSN: 1367-4803, DOI: 10.1093/bioinformatics/btu010
- BOLSTAD B M ET AL: "A COMPARISON OF NORMALIZATION METHODS FOR HIGH DENSITY OLIGONUCLEOTIDE ARRAY DATA BASED ON VARIANCE AND BIAS", BIOINFORMAT, OXFORD UNIVERSITY PRESS, SURREY, GB, vol. 19, no. 2, 22 January 2003 (2003-01-22), pages 185-193, XP008041261, ISSN: 1367-4803, DOI: 10.1093/BIOINFORMATICS/19.2.185
- Anonymous: "Two-Color Normalization", , 27 February 2015 (2015-02-27), XP055411795, Retrieved from the Internet: URL:https://web.archive.org/web/2015022711 4219/https://discover.nci.nih.gov/microarr ayAnalysis/Two.Color.Preprocessing.jsp [retrieved on 2017-10-02]

## Description

Embodiments of the present invention refer to a method for performing the normalization of the intensity values obtained to perform sequencing analysis. Additional embodiments refer to a corresponding computer program and the corresponding apparatus.

Document C. YE ET AL: "BlindCall: ultra-fast base-calling of high-throughput sequencing data by blind deconvolution", BIOINFORMATICS., vol. 30, no. 9, 9 January 2014 (2014-01-09), pages 1214-1219, ISSN: 1367-4803, DOI: 10.1093/bioinformatics/btu010 discloses normalizing intensity values obtained for sequencing by defining parametrized distributions for the intensity values for each channel and applying transfer functions to the intensity values of each channel so that the parametrized distributions are mapped to a common distribution which is a obtained by combining the parametrized distributions.

Next generation sequencing (NGS) characterizes the sequence of millions of DNA molecules in parallel. To this end, millions of DNA molecules are immobilized randomly at different positions on an imaging surface and copied to form local clusters of clonal DNA molecules. Sequencing of these template molecules is performed by synthesis of complementary DNA, incorporating fluorescently labeled nucleotides with 4 distinct fluorescent dyes for each specific nucleotide (A, C, G, and T). Specific sequencing chemistry ensures that each sequencing iteration (cycle) incorporates only one nucleotide at a time. For each cycle the sequencing apparatus (sequencer) takes images with four distinct filter settings (channels), one for each nucleotide specified wavelength specified by the respective fluorescent dyes. The entire sequencing run is therefore represented by a set of *n* x *c* images, where n equals the number of channels (typically 4) and c equals the number of cycles.

This set-up enables deduction of the nucleotide-sequence of the template DNA molecules from the sequence of images acquired for all channels for each sequencing cycle: Given that the position of a template DNA cluster is known, the intensity profiles over all channels at this given position allows deduction of the incorporated fluorescently labeled nucleotide (base-calling). In theory non-zero intensities should be detectable in only one channel which resembles the nucleotide present in the template DNA. A variety of factors including imaging and sequencing noise result in a derivation from this optimal case: non-zero intensity values are typically observed for all channels. Performing base-calling for all sequencing cycles for a given position allows deduction of the full nucleotide sequence (read).

As indicated above, the signal distribution among the channels determines base-calling. However, deviations from the optimal case (i.e. only one channel is characterized by non-zero intensity values) can be ascribed to two components:
i) bias; and
ii) noise.

While the first can be described as a systematic offset from zero, the latter can be described by fluctuation around this offset from sample to sample. Importantly, these components can be channel-specific and thus may lead to biased base-calling. Several channel-specific bias introducing phenomena are known and are typically corrected in the algorithm chain leading to base-calling. These include background correction and crosstalk correction. However, only known phenomena can be corrected using such a model based approach. Therefore, there is the need for an improved approach.

It is an objective of the present invention to provide a concept for base-calling or especially the post image analysis used for base-calling procedures having a reduced impact to biasing and noise effects.

This objective is solved by the subject matter of the independent claims.

Embodiments of the present invention provide a method for performing a normalization of intensity values obtained to perform a sequencing analysis. The method comprises the four basic steps of "receiving a plurality of image data for a plurality of channels", "parametrization of an intensity distribution over all or a subset of all positions for the plurality of channels", "combining parametrized distributions for the plurality of channels" and determining for each of the plurality of channels a transfer function". The image data comprise a plurality of images (e.g. generated by a sequencing apparatus). Each received image describes for the respective channel, for example for the four channels A, C, G, and T (belonging to four bases), an intensity distribution in terms of a spatial light density distribution over all positions or at least over a subset of all positions of the respective image. The intensity distribution is parametrized to obtain a parametrized distribution for each of the plurality of channels. Starting from the parametrized distributions for the plurality of channels, the parametrized distributions are combined with the aim to obtain a common distribution for all or for at least two of the plurality of channels. The last step of determining the transfer function for each channel is performed such that the respective transfer function for the respective channel maps the corresponding intensity distribution to the common distribution.

Teachings disclosed herein are based on the finding that the intensity distribution is typically channel-specific leading to systematic differences between the channels. However, since over the entire image, the number of A-, C-, G-, and T- base-calls should be randomly equally distributed, the overall intensity level of all channels should be comparable to each other. In order to maintain the comparableness, all or the relevant channels can be adapted with regard to its intensity level. This is achieved when collapsing each channel-specific intensity distribution into one common distribution and determining corresponding correction factors for the respective channel such that same can be mapped to the (collapsed) common distribution, afterwards. The usage of the correction factor for the respective channel produces unbiased intensity profiles over all channels and leading to unbiased base-calling (without or with reduced systematic differences). Here, it is beneficial that the correction of the base-calling can be performed without knowing the exact phenomenon leading to the biasing.

According to another embodiment, the step of parametrization is performed using the substep of maximum-likelihood estimation, maximum-a-posteriori-estimation, determining a summary statistic for one or more or all channels or determining a specific parameter set describing an intensity distribution (e.g. the maxima and minima of said distribution) for one or more or all channels. The estimation for determining procedures enables beneficially to parametrize different intensity distributions, such that the same are comparable to each other.

According to embodiments a distinction between two types of intensity distributions is made, namely non-normal distributions and normal distributions. Commonly, the type of intensity distribution has an impact to the type of the transfer function, so that typically, a non-linear transformation is used for non-normal distributions, wherein a linear transformation is used for normal distributions. For example, the parametrized distribution for one or more or all of a plurality of channels is described by a Gaussian distribution by a distinct mean and a distinct standard deviation. Here, the respective transfer function for the respective channel can also be described by a Gaussian distribution comprising the mathematic operations for subtracting the mean from the corresponding intensity distribution and/or dividing the corresponding intensity distribution by the standard deviation. According to another embodiment transfer functions like a log-transformation, a square-root transformation, a Box-Cox transformation or a Yeo-Johnson transformation can be used for non-normal intensity distributions. Alternatively, another transformation enabling to transform non-normal distribution to an approximated normal distribution can be applied. Since the intensity distributions for different sequencing analysis techniques or procedures vary with regard to its type, it is beneficial to have different transformation approaches enabling to handle the different types of intensity distributions such that the normalization process can be applied to each used case.

Although the above embodiments suggest performing the normalization over the different channels within one cycle of a sequencing analysis, it should be noted that according to further embodiments the parametrization, combining and determining is performed for a plurality of cycles. In this case, the method comprises the step of determining the transfer functions such that same comprise smoothing functions. Here, smoothing means that the transfer function smoothes the intensity distribution over the multiple cycles such that there are no jumps from cycle to cycle, i.e. the smoothing function is selected such that same describes a maximum change of the intensity value over the number of cycles and/or such that the intensity value within at least two subsequent cycles remain on a constant level. This case depends on the assumption that channel-specific distributions of subsequent cycles are similar and thus their characterization parameters (i.e. distribution parameters or summary statistics) are correlated. According to another variant the normalization over a plurality of cycles may be performed such that a normalization over all cycles of the analysis can be done. Here, the respective transfer function for the respective channels comprise a normalization function enabling that the intensity values of the plurality options are normalized over the number of cycles for all channels and/or such that the intensity values remain on an average constant level over the number of cycles. This approach enables to correct effects like an intensity drift. However, in case there is a trend which causes decreasing or increasing intensity values of the number of cycles this approach may have a negative effect to the sequencing analysis. Therefore, the normalization function is determined just in the case if no Trend of the one or more channels over the number of cycles is expected or detected. This trend detection may be performed based on the image data before applying the transfer function to the plurality of channels and cycles.

Of cause, it is possible to perform the normalization (parametrization, combining and determining of the common target distribution) over the different channels and different cycles. Thus, according to another embodiment the basic method may be performed differently, namely such that the step of receiving a plurality of image data and prioritization of the intensity distribution is performed for a plurality of channels and cycles, wherein the step of combining and determining is performed for the plurality of cycles. Expressed in other words this means that the transfer function enables a mapping over a plurality of cycles instead of a mapping over a plurality of channels. Here, the principles as discussed in context of this moving function may be applied for at least one channel. As described above, the combination of normalizing the intensity distribution over cycles and channels is also possible.

The above embodiments start from the assumption that preferably (but not necessarily) all of the channels are mapped to a common distribution function. However, especially in case when a smoothing over a plurality of cycles is performed, it may be beneficial that a common distribution function is determined for each channel or for at least two channels independently. Here, the common distribution function for all of the plurality of channels may be described by a set of functions comprising for each channel a common distribution function, wherein at least two of the common distribution functions differ from each other. Each respective transfer function is determined such that the respective transfer function for a respective channel maps the corresponding intensity distribution to the corresponding common distribution function of the channel. As mentioned above, this approach having a plurality of common distribution functions is selected, if a smoothing over a plurality of cycles is performed. Here, the plurality of common distribution functions may be used beneficially if a trend of one or more channels over the number of cycles is expected or detected.

According to another embodiment a computer readable digital storage medium having stored thereon a computer program having a program code which, when executed on a computer, causes the computer to carry out the one of the above methods,
According to another embodiment an apparatus tor performing a normalization or intensity values obtained to perform a sequencing analysis is provided. Here, the apparatus comprises an interface and a processor. The interface receives the plurality of image data, wherein the processor performs the parametrization, combination and determining of the transfer functions.

Embodiments of the present invention will subsequently be discussed referring to the enclosed figures, wherein:
- Fig. 1a: shows a flowchart illustrating the method for performing a normalization of intensity values belonging to image data used for sequencing analyses, wherein the normalization is performed over a plurality of channels (one cycle) according to an embodiment;
- Fig. 1b: shows a flowchart illustrating the method for normalization of the intensity values, wherein the normalization is performed over a plurality of cycles according to another embodiment;
- Fig. 1c: shows a schematic representation of image data belonging to different channels and different cycles for illustrating the principle of sequencing analysis;
- Figs. 1d-1e: show schematic diagrams illustrating the intensity differences between the single channels used for the sequencing analysis;
- Fig. 1f: shows schematic diagrams of intensity distributions for illustrating the improvements achieved by the normalization according to embodiments;
- Figs. 2a-c: show schematic diagrams illustrating intensity distributions and normalization thereof according to an embodiment; and
- Figs. 3a-c: show schematic diagrams of intensity distribution-characterization-parameter for illustrating the principle of performing the normalization over a plurality of cycles.

Below, embodiments will subsequently be discussed in detail referring to the enclosed figures. Here, identical reference numerals are provided to elements on method steps having similar or identical functions so that the description thereof is mutually applicable and interchangeable.

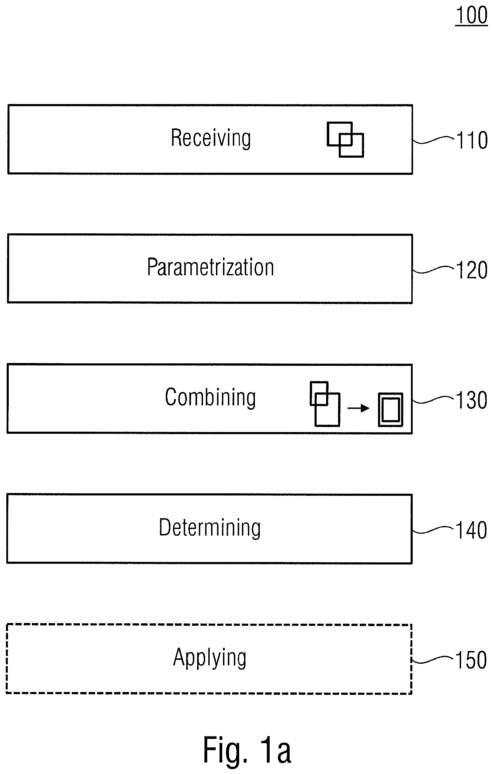

Fig. 1a shows the basic method 100 for performing a normalization of intensity values. This method 100 is mainly used for sequencing analysis. For sequencing analysis or DNA analysis single-stranded DNA fragments of the template molecules are extended such that the single basis or nucleotides (in general molecules of interest) can be detected using a fluorescent dye.

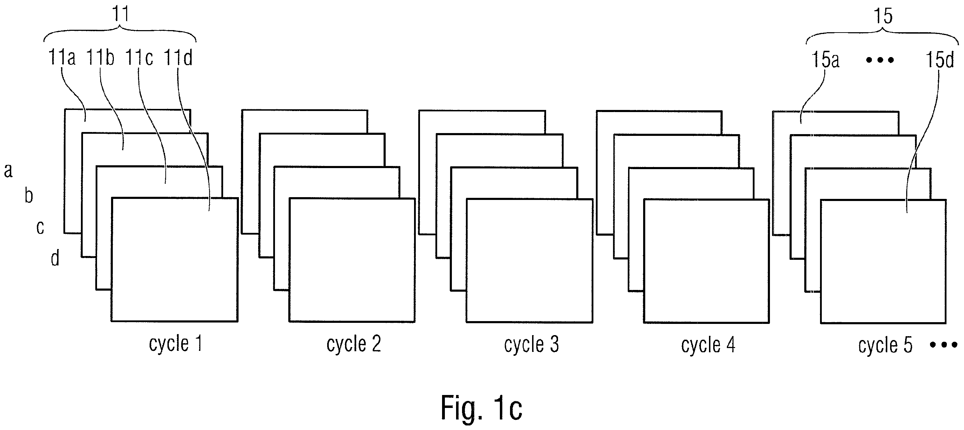

This is exemplarily illustrated by Fig. 1c. Fig. 1c shows an exemplary set of images generated by a sequencing apparatus. For each sequencing cycle 11 to 15 a set of *n* images 11a to 11d/11a to 15d is acquired, one for each channel (belonging to specified fluorescent dye). Note that in some images the channels A to D are referred to A, C, G and T, which is the official labelling used for (DNA) sequencing. The intensity values are encoded. While white pixel may represent emitted light by fluorophores, dark pixel areas may represent background intensities.

Typically a distinction is made between four nucleotides (A, C, G and T), such that four distinguishable fluorescent dyes are used. Each channel can be detected using an own channel. The channels can be analyzed using different filter settings, so that a plurality of images 11a to 11d has to be analyzed during one cycle. Each cycle refers to a sequencing iteration (cf. cycle 11, cycle 12, cycle 13, etc., cycle 15, etc.). Background thereof is that during each sequencing iteration/cycle 11 to 15, just one nucleotide can be detected. Just for the sake of completeness, it should be noted that between the single cycles 11 to 15 a procedure comprising cleaving the fluorescent dye and extending the sequencing primer is performed such that a new base/nucleotide can be incorporated.

Since for each sequencing cycle 11 to 15 a set of *n* images (11a-11d) are required. The entire sequencing run is represented by a set of *n* x *c* images 11a to 15d, where *n* equals the number of channels (typically four) and *c* equals the number of cycles (here five). Expressed in other words, this means that each sequencing run has two main dimensions, namely the dimension defined by the number of cycles and the dimension defined by the number of channels.

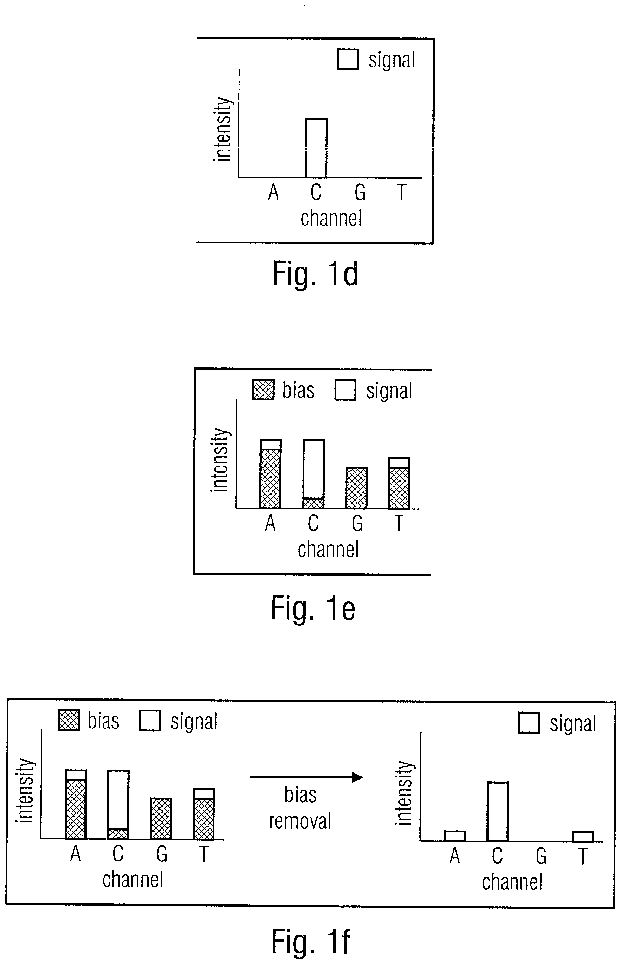

As indicated above, the signal distribution among the channels a-d (A, C, G and T) determines base-calling. In the optimal case just one channel is characterized by non-zero intensity values. This optimal case is shown by Fig. 1d. As illustrated by Fig. 1d just the channel c outputs a signal. In such a case it can be assert that within the examined cycle at the examined position of the image a nucleotide of the type c is detected.

However, in reality the measured intensity signal is distorted by biasing and noise components. Such biased intensity values are illustrated by Fig. 1e. Here, the biasing and the signal component of each signal belonging to a respective channel are marked by different hatchings. Typically, these components lead to a systematic offset from zero within the specific channels. Due to the biasing the intensity values for the channels a and d are nearly the same. Therefore, the two channels cannot easily be distinct from each other.

The method 100 enables to remove the channel-specific contribution to bias and/or noise by performing a normalization. Here, the data driven approach enables to normalize intensity values over all channels A to D (preferably within one cycle 11, 12, etc. or 15). Within the first step 110 the plurality of image data for the plurality of channels, i.e., for the example of Fig. 1C, the images 11a to 11a are received, Each image 11a to 11d describes, for the respective channel A to D, an intensity distribution over all positions of the respective image element 11a to 11d or, at least over a subset of all positions, when just a portion of each image 11a to 11d should be analyzed. After that, all image data for the first cycle 11 are available.

The intensity distributions over the relevant positions of the images 11a to 11d, or of at least for two images, are parametrized in order to obtain a parametrized distribution for the plurality of channels. This step is marked by the reference numeral 120. The parametrized distribution can, for example, be a summary statistic. It is described using a specific parameter, namely the mean. Therefore, the step 120 can comprise the sub-step of determining a significant parameter or parameter set describing the behavior of the respective channel, like the summary statistic. Alternatively, a maximum-likelihood estimation or aposteriori-estimation can be used.

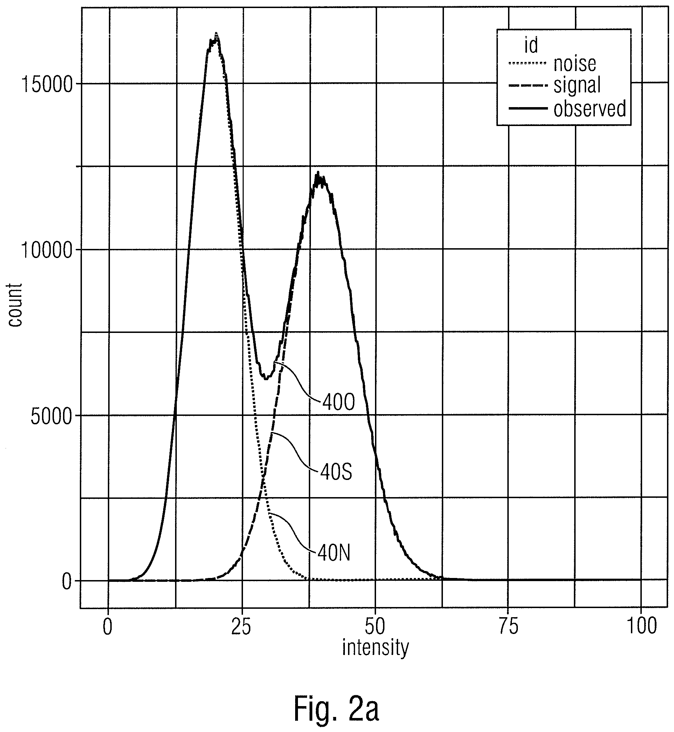

Here it should be noted that the intensity distribution over all positions and/or the parametrized distributions describe, for example, the number of counts in correlation to respective intensity values for the respective channel of the plurality of channels a-d (cf. Fig 2a). Thus, the intensity distribution is defined within a two dimensional space.

Within the next step 120, the plurality of the parametrized distributions for all or at least two channels a to d (A, C, G and T) are combined such that a common distribution for all or the relevant channels within the first cycle can be obtained. The common distribution is an average of all or all relevant parametrized distributions.

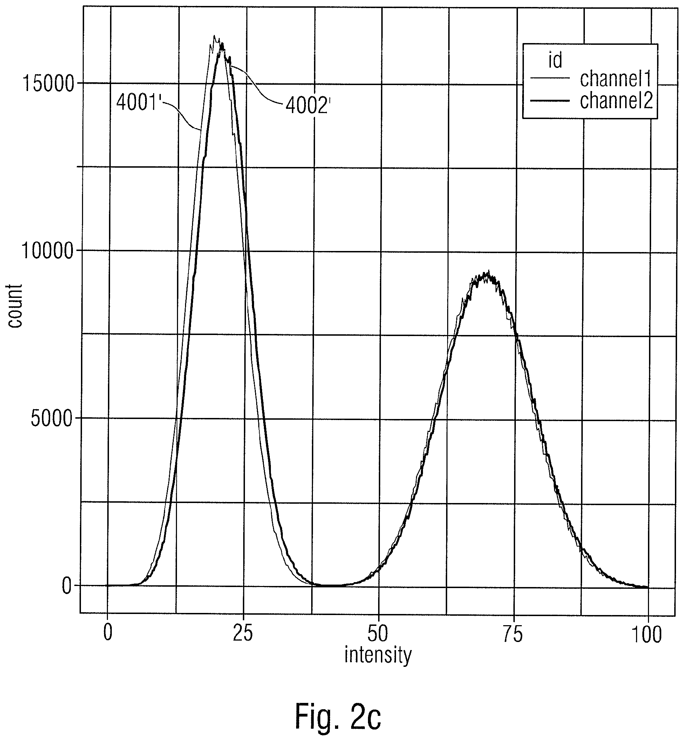

In context of the common distribution it should be noted that the common distribution (for at least two, all relevant or all channels a-d) represents an average of (at least two,) all relevant (, or all) parametrized distributions / intensity distributions of the plurality of channels. For example, the common distribution for (at least two,) all relevant (or all) channels may describe an averaged number of counts in correlation to an averaged intensity values for at least two, all relevant or all channels a-d (cf. Fig. 2c). Consequently, the common distribution or each point within the common distribution may be defined by at least two parameters (number of counts and intensity), e.g. derived from the parametrized distributions. Consequently, the common distribution is also defined within a two dimensional space.

Starting from the common distribution, the intensity values of the respective channels 11a to 11d are mapped to the common distribution using a respective transfer function for each channel 11a to 11d. This step is marked by the reference numeral 140. The respective transfer functions can be used for filtering or normalizing the images 11a to 11c. Since the respective transfer function is determined based on all or at least all relevant positions (a sub-set of all positions) of the respective channel 11a to 11d, the transfer function enables the channels 11a to 11d to have - an averaged - same behavior, so that channel specific effects can be avoided or eliminated.

Example: starting from a basic example, merely that one channel has a substantially higher brightness than another channel it is clear that the determined transfer function enables dimming of the entire bright channel, especially, each intensity value of the single position within the channel. Due to the dimming (application of the channel specific transfer function, the intensity values belonging to the signal positions within the channels are more comparable than without applying the transfer function or normalization procedure.

If now, the image analysis of the respective channel 11a to 11d or, in more detail, of each relevant position (sub-set of all positions) or at least of one position of the respective channel 11a to 11d, is performed while applying the respective transfer function to the intensity value, the intensity values of the different channels 11a to 11d are distinguishable from each other. This is illustrated by Fig. 1f.

Fig. 1f shows in the left-hand diagram the measured intensity values of Fig. 1f. The application of the respective transfer function to the respective channel enables removal of the bias, such that an intensity values (for the relevant positions / the one position) within the four channels can be achieved, which approximately equates to the intensity diagram as discussed with respect to Fig. 1d. Here, it is important that this bias cannot be extracted directly from the base call but has to be estimated via the intensity value distributions over all base calls as described above. Due to the application of the transfer function, the channel specific biases are removed by filter normalization and, allowing unambiguous base-calling to channel C.

The application of the channel specific transfer function to the intensity values is an optional step which is marked by the reference numeral 150. The usage of the method steps 110 to 140 enables determination of the transfer functions, wherein the performing of all method steps 110 to 150 enables the normalization of the channels within one cycle. Therefore, starting from a basic approach, the step 150 is an optional step.

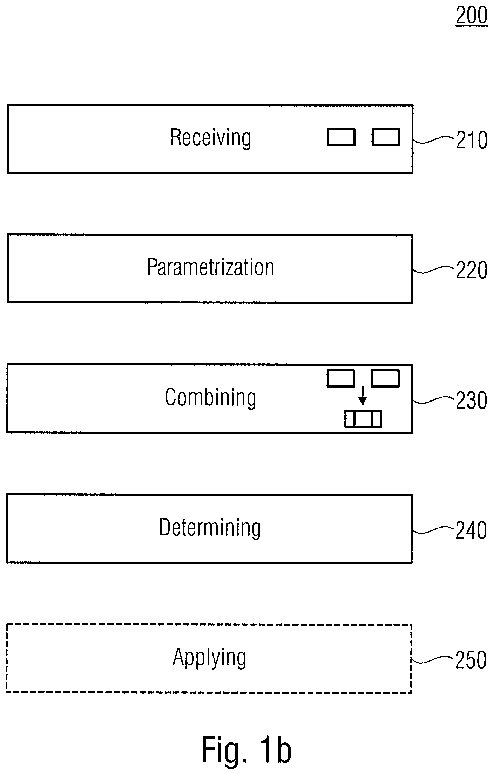

Although in embodiments the normalization has been described in context of normalizing different channels to each other, the normalization can also additionally or alternatively be performed such that one or more channels are normalized over the plurality of cycles. This approach is illustrated by Fig. 1B showing a block diagram of method 200. Method 200 comprises the basic steps 210 to 240 and the optional step 250. The step 210 is comparable to the step 110, wherein the image data does not only comprise the images of one cycle 11, but also the images belonging to a plurality of cycles or all cycles. Within the step 220 the intensity distributions over all positions or over all relevant positions of the received images are parametrized. After that, the parametrized distributions, at least for one channel over the plurality of cycles, are combined in order to obtain a common distribution for at least one channel over the plurality of cycles. The step is marked by the reference numeral 230. This step may optionally be performed such that a common distribution for plurality of cycles and channels is obtained. Starting from the common distribution, the determining of the respective transfer function for the at least plurality of cycles 11 to 15 is performed within the step 240. The step 250 is the optional step of applying the determine transfer functions during the image analysis.

With respect to Figs. 2A and 2C, the background of the above method will be described.

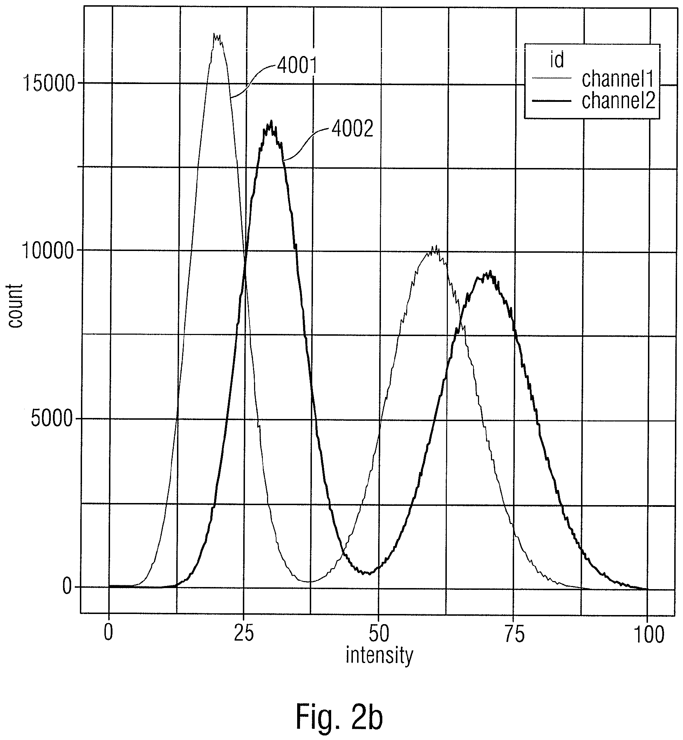

Fig. 2A shows a diagram illustrating the number of counts (y axis) for respective intensity values (plotted over the x axis). Here, the observed values are marked by the reference numeral 40O. This observed signal 40O results from two signal portions, namely the noise 40N and the real signal 40S. This schematic representation of the intensity distribution for a given channel over all positions makes it clear that the observed intensity distribution 40O is the result of positions with intensities drawn from the noise distribution 40N and those drawn from the signal distribution 40S. Dependent on the separation of noise and signal distribution, the observed distribution may or may not be bimodal. The diagram can be described with other words, in that the intensity values 40O are observed in a given channel can be modelled by a random variable. For each position the random variable is drawn from one of two distinct distributions, namely "signal distribution" 40S in case the channel indicates an incorporated nucleotide match and, "noise distribution" 40N, otherwise. The observed intensity-distribution over all positions will be characterized by the combination of both distributions. The intensity distribution may be channel-specific, leading to systematic differences as can be seen by Fig. 2B.

Fig. 2B shows a schematic representation of intensity distributions for two channels 4001 and 40O2. Here, the observed intensity distributions 4001 and 40O2 are distinct for both channels. This difference may lead to biased-based calling.

By the usage of the channel specific transfer functions, the observed intensities over all positions 4001 and 40O2 can be distorted such that the same follow a common distribution. This means that the goal of the approach is to collapse all channel specific intensity distributions into one common distribution, thereby producing unbiased intensity profiles over all channels, leaving two unbiased-base calling. This collapse may be done via means of the channel specific transfer functions determined using the method 100. The result is shown by Fig. 2C.

Fig. 2C shows a schematic representation of corrected intensity distribution for two channels (corresponding to the common distribution). Here, the corrected intensity diagrams are marked by the reference numeral 40O1' and 40O2'. This collapse of the channel specific intensity distributions 40O1' and 40O2' may lead to unbiased-base calling.

Here, the observed intensity values of all positions within the respective channels are distorted using the transfer function, such that the intensity distributions 40O1' and 40O2' are achieved.

Starting from the normalization between two or more channels, a preferred way to determine the respective transfer functions enabling collapse of all channel-specific intensity distribution into one distribution will be discussed. For this, the observed intensity distributions have to be characterized.

In order to collapse all channel specific intensity distributions into one common distribution, the observed intensity-distributions have to be characterized. Distribution characterization can be classified into two approaches: i) parametrization of a defined distribution and ii) characterization of an undefined distribution. The first case applies if the phenomena leading to noise and signal are known and the resulting family of observed distribution can be derived. In this case parametrization can be performed by probabilistic modeling and standard procedures like maximum-likelihood-estimation or maximum-aposteriori-estimation. If no probabilistic model of the observed intensity distribution is known, characterization may occur via summary statistics. Depending on the complexity of the underlying distribution and how distinct the channel specific distributions are this can be performed by a single summary statistic or a combination of summary statistics. Common applicable summary statistics include mean, mode, standard deviation (SD) as well as order statistics.

Given the set of characterized intensity distributions for all channels, the second step is to find a common distribution that all channel-specific distributions can be collapsed to. This is performed by channel-specific transformations of the data such that the resulting intensity distributions follow a distribution characterized by specified parameters or summary statistics. The nature of the transformation depends on the underlying distribution and may be a simple linear transformation, a non-linear transformation, a set of linear or non-linear transformations or a combination of both sets. In the simplest case, intensity distribution may be described by a Gaussian distribution. In this case channel specific distributions can be collapsed by normalizing the Gaussian distribution by subtraction of the mean and division by SD.

Analogously, instead or additionally, to the intensity distribution normalization between plurality of channels as cycle-specific normalization can be used.

The intensity normalization approach described above may be applied to all cycles or for each cycle individually. The latter case may be desirable to correct for differences introduced as a function of sequencing cycle (i.e. drift). Drift may occur for example in the case of cycle specific sequencing configuration (i.e. adapted imaging or chemistry) or due to decaying or increasing performance. The cycle-specific normalization can be performed as described above. Alternatively, the cycle context can be used to smooth normalization transformations. This case depends on the assumption that channel-specific distributions of subsequent cycles are similar and thus their characterization parameters (i.e. distribution parameters or summary statistics) are correlated. For smoothing, channel specific distribution characterization is performed as described above for each cycle or groups of cycles. Smoothing may be performed by a variety of functions including sliding window approaches (mean, median, Gaussian filter, local model fitting) or model fitting on the entire cycle set (e.g. polynomials). To estimate the transformation model to the specified target distribution, the parameter estimated by the smoothing function is used instead of the parameter derived from the distribution characterization.

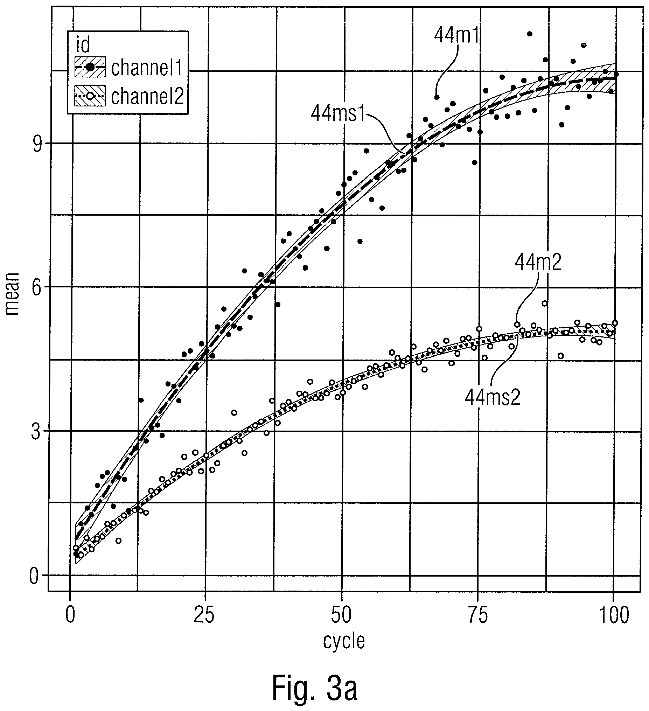

This approach will be discussed with respect to Figs. 3A to 3C.

Fig. 3A shows a schematic representation of one distribution-characterization-parameter, here, the mean. The mean, as an example of a summary statistic, can be used to characterize the intensity distribution for the two channels. This parameter for the two channels is marked by the reference numeral 44M1 and 44M2. The mean is determined for each cycle individually, as indicated by the dots, and plotted versus the cycle number. As can be seen, the subsequent means are correlated, but show significant noise. The noise can be removed which results in the smoothed line. After noise removal, the mean retains significant differences between both channels indicating a systematic difference which may lead to biased-base calling. The smoothed line may then be used instead of the noisy cycle-specific values, e.g. for the channel specific normalization.

In order to eliminate the systematic differences, the distributions can be normalized using the above-discussed approach according to which the plurality of channels is distorted using the transfer functions such that each channel is mapped to a common distribution. The transformation can be performed such that the general trend of the parameter versus cycle is retained.

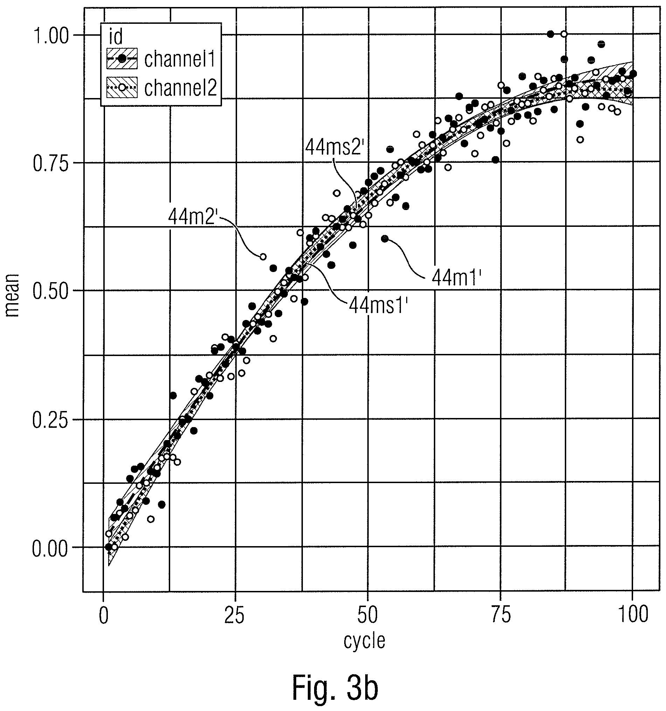

This approach is illustrated by Fig. 3B showing the distorted means 44M1' and 44M2' of the two channels together with the smoothed means 44MS1' and 44MS2'. The distorted values 44M1' and 44M2' and 44MS1' and 44MS2' leave the overall trend of the mean with respect to the cycle intact, but normalizes the distributions for each cycle such that the mean is similar for both channels. Here, the target distribution is selected such that the mean is scaled between 0 and 1. This can be achieved by the definition of the target distribution and the derivation of the transformation to reach this distribution. If the general trend should be retained the defined target distribution should reflect this. If the trend is the same, for all channels collapsing can be achieved by normalizing the characterization parameters between defined values as shown here (min=0, max=1).

Expressed in other words, this means that according to an embodiment, a normalization between the single channels together with a smoothing can be used. Due to the interchannel-normalization, the distinction between the same is improved wherein the smoothing enables avoidance of jumps between subsequent cycles.

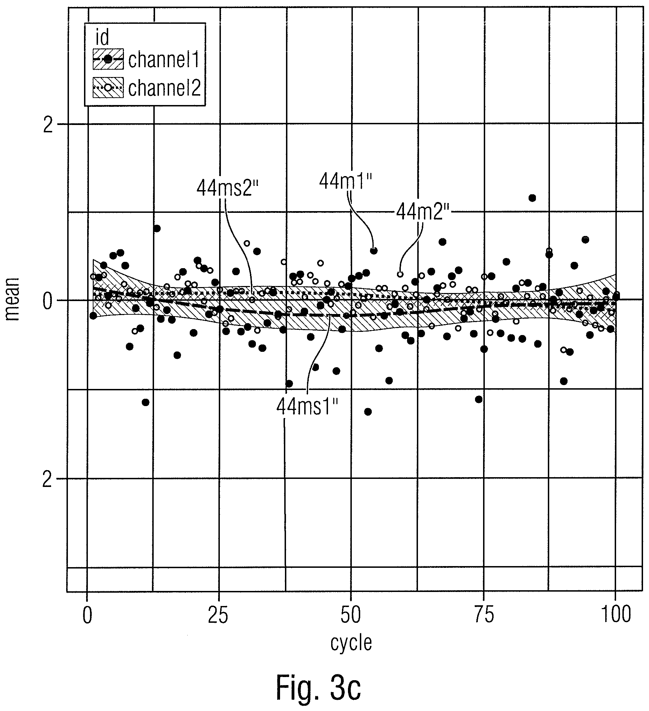

According to another embodiment, a further approach normalizes both the channels with respect to the other channels and the radius over all cycles such that the parameter profiles are stationary with respect to the plurality of cycles. This approach is illustrated by Fig. 3C. Fig. 3C shows the means for the two channels 44M1" and 44M2" together with the smoothed versions thereof, 44MS1" and 44MS2". As can be seen, this second approach enables generation of stationary distributions over the entire sequencing run. This is achieved by the definition of the target distribution which should be identical for all cycles. The obtained transfer function for the respective channel can be used to normalize the channels during / for performing the sequencing analysis.

Below, an enhanced example will be discussed. Let us assume that we measure intensities in 4 distinct channels and that for each cycle the channel-specific intensity values are distributed according to a Gaussian distribution with distinct mean, but identical SD. In this case the intensity-distribution normalization is simple: i) The channel-specific distribution for each cycle is characterized by the mean of the intensities. ii) Mean versus cycle is determined for each channel individually and smoothed using an appropriate method (e.g. windowed mean). iii) A target distribution for each cycle is determined. Let us further assume that the mean of intensities increases with respect to cycles with a similar function for all channels and that we want to retain this general trend. To retain the mean versus cycle trend, the mean of the target distribution is specified such that it ranges from 0 to 1 and follows the overall trend. iv) Transformation is performed by subtracting the smoothed observed channel mean and adding the mean of the target distribution.

Other examples of collapsing channel intensity distribution may include normalizing Gaussian distributions with distinct mean and standard deviation via standardization (i.e. mean = 0, SD = 1) or transformation of non-normal distribution to approximate normal distributions (e.g. log-transformation, square-root transformation, Box-Cox transformation, Yeo-Johnson transformation) followed by the standardization of the resulting approximately normal distribution.

Although, referring to the above discussion, all embodiments have been described in the context of a method it should be noted that the idea may also be implemented as an apparatus. Here, all the above implementation aspects may also be used in context of the apparatus.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus. Some or all of the method steps may be executed by (or using) a hardware apparatus, like for example, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a digital storage medium, for example a floppy disk, a DVD, a Blu-Ray, a CD, a ROM, a PROM, an EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may for example be stored on a machine readable carrier.

Other embodiments comprise the computer program for performing one of the methods described herein, stored on a machine readable carrier.

In other words, an embodiment of the inventive method is, therefore, a computer program having a program code for performing one of the methods described herein, when the computer program runs on a computer.

A further embodiment of the inventive methods is, therefore, a data carrier (or a digital storage medium, or a computer-readable medium) comprising, recorded thereon, the computer program for performing one of the methods described herein. The data carrier, the digital storage medium or the recorded medium are typically tangible and/or non-transitionary.

A further embodiment of the inventive method is, therefore, a data stream or a sequence of signals representing the computer program for performing one of the methods described herein. The data stream or the sequence of signals may for example be configured to be transferred via a data communication connection, for example via the Internet.

A further embodiment comprises a processing means, for example a computer, or a programmable logic device, configured to or adapted to perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

## Claims

1. A computer implemented method (100) for performing a normalization of intensity values obtained to perform sequencing analysis, the method (100) comprises the steps:
receiving (110) a plurality of image data (11a-11d, 15a-15d) for a plurality of channels (a-d), each image data (11a-11d, 15a-15d) describes for the respective channel (a-d) an intensity distribution over all positions of an image of the plurality of image data (11a-11d, 15a-15d);
parametrization (120) of the intensity distribution over all positions for the plurality of channels (a-d) to obtain parametrized distributions for the plurality of channels (a-d); wherein the parametrized distributions for the plurality of channels (a-d) is described using the mean of the intensity values over all positions for the plurality of channels (a-d);
combining (130) the parametrized distributions for the plurality of channels (a-d) to obtain a common distribution for all of the plurality of channels (a-d); wherein the common distribution represents an average of all parametrized distributions of the plurality of channels; and
determining and applying (140) for each of the plurality of channels (a-d) a transfer function such that the respective transfer function for the respective channel (a-d) maps the corresponding intensity distribution to the common distribution.

2. The method (100) according to claim 1, wherein the parametrization (120) and
determining (140) is performed for four channels (a-d), each belonging to a specific nucleotide, and/or
wherein the parametrization (120) and determining (140) is performed for four channels (a-d) each filtered to determine one of four distinct fluorescent dyes marking four specific nucleotides.

3. The method according to claim 1 or 2, wherein each position within the plurality of channels (a-d) belongs to its own sequence and/or to its own DNA sequence.

4. The method (100) according to one of the previous claims, wherein the step of parametrization (120) is performed using the substep of maximum-likelihood-estimation, maximum-aposteriori-estimation, determining (140) a summary statistic for one or more or all channels (a-d) or determining (140) a specified parameter set describing an intensity distribution of one or more or all channels (a-d).

5. The method (100) according to one of the previous claims, wherein the respective transfer function enables a linear transformation of the corresponding intensity distribution to the common distribution.

6. The method (100) according to one of claims 1 to 5, wherein the respective transfer function enables a non-linear transformation of the corresponding intensity distribution to the common distribution.

7. The method (100) according to one of the previous claims, wherein the intensity distribution for one or more or all of the plurality of channels (a-d) is described by a Gaussian distribution with a distinct mean and a distinct standard deviation.

8. The method (100) according to claim 7, wherein the respective transfer function for the respective channel (11a-11d) described by the Gaussian distribution comprises the mathematic operations of subtracting the mean from the corresponding intensity distribution and/or dividing the corresponding intensity distribution by the standard deviation.

9. The method (100) according to claim 6, wherein the intensity distribution for one or for more or for all of the plurality of channels (a-d) is described by an non-normal distribution.

10. The method (100) according to claim 9, wherein the respective transfer function enables a log-transformation, a square-root transformation, a Box-Cox transformation or a Yeo-Johnson transformation.

11. The method (100) according to one of the previous claims, wherein the parameterization (120), combining (130) and determining (140) is performed for each of a plurality of cycles individually.

12. The method (100) according to one of claims 1 to 10, wherein the parametrization (120), combining (130) and determining (140) is performed for a plurality of cycles (11-15) or all cycles (11-15).

13. The method (100) according to claim 12, wherein the step of determining (140) the transfer function is performed such that the respective transfer function for the respective channel (a-d) comprises a smoothing function;
wherein the smoothing function describes a maximum change of the intensity value over the number of cycles (11-15) and/or the smoothing function is determined (140) such that the intensity value is smoothed over the number of cycles (11-15).

14. The method (100) according to claim 12 or 13, wherein each respective transfer function comprises a normalization function;
wherein the normalization function is determined (140) such that the intensity values of the plurality of channels (a-d) are normalized over the number of cycles (11-15) for all channels (a-d).

15. The method (100) according to claim 14, wherein the respective normalization function is determined (140) if no trend of one or more channels (a-d) over the number of cycles (11-15) is detected.

16. The method (100) according to claim 12, wherein the common distribution for all of the plurality of channels (a-d) is described by a set of functions comprising for each channel a common distribution function, wherein at least two of the common distribution functions differ from each other;
wherein each respective transfer function is determined (140) such that the respective transfer function for the respective channel (a-d) maps the corresponding intensity distribution to the corresponding common distribution function.

17. The method (100) according to claim 15, wherein the plurality of common distribution functions is determined (140) if a trend of one or more channels (a*-*d) over the number of cycles (11-15) is detected.

18. The method (100) according to claim 16 or 17, wherein each transfer function comprises a smoothing function, wherein the smoothing function describes a maximum change of the intensity value over the number of cycles (11-15) and/or wherein the smoothing function is determined (140) such that the intensity value used for the respective channel (a-d) is smoothed over the number of cycles (11-15).

19. The method (100) according to one of claims 1 to 18, wherein the intensity distribution over all positions and/or the parametrized distributions describe the number of counts in correlation to respective intensity values for the respective channel of the plurality of channels (a-d); and/or
wherein the common distribution for at least two, all relevant or all channels (a-d) represents an average of at least two, all relevant, or all parametrized distributions of the plurality of channels and/or wherein the common distribution for at least two, all relevant or all channels describe an averaged number of counts in correlation to an averaged intensity values for at least two, all relevant or all channels (a-d).

20. A computer readable digital storage medium having stored thereon a computer program having a program code which, when executed on a computer, cause the computer to carry out the method according to one of the previous claims.

21. Apparatus for a normalization of intensity values obtained to perform sequencing analysis, comprising:
an interface for receiving a plurality of image data (15a-15d) for a plurality of channels (a-d), each image data (11a-11d, 15a-15d) describes for the respective channel (a-d) an intensity distribution over all positions of the image of the plurality of image data (11a-11d, 15a-15d);
a processor configured to parametrize the intensity distribution over all positions for the plurality of channels (a-d) to obtain parametrized distribution for the plurality of channels (a-d) and configured to combine the parametrized distribution for the plurality of channels (a-d) to obtain a common distribution for all of the plurality of channels (a-d); wherein the parametrized distributions for the plurality of channels (a-d) is described using the mean of the intensity values over all positions for the plurality of channels (a-d); wherein the common distribution represents an average of all parametrized distributions of the plurality of channels; and ,
wherein the processor is configured to determine and apply (140) for each of the plurality of channels (a-d) a transfer function such that the respective transfer function for the respective channel (a-d) maps the corresponding intensity distribution to the common distribution.

## Patentansprüche

1. Ein computerimplementiertes Verfahren (100) zum Durchführen einer Normierung von Intensitätswerten, die erhalten werden, um eine Sequenzierungsanalyse durchzuführen, wobei das Verfahren (100) folgende Schritte aufweist:
Empfangen (110) einer Mehrzahl von Bilddaten (11a-11d, 15a-15d) für eine Mehrzahl von Kanälen (a-d), wobei alle Bilddaten (11a-11d, 15a-15d) für den jeweiligen Kanal (a-d) eine Intensitätsverteilung über alle Positionen eines Bildes der Mehrzahl von Bilddaten (11a-11d, 15a-15d) beschreiben;
Parametrisierung (120) der Intensitätsverteilung über alle Positionen für die Mehrzahl von Kanälen (a-d), um parametrisierte Verteilungen für die Mehrzahl von Kanälen (a-d) zu erhalten; wobei die parametrisierten Verteilungen für die Mehrzahl von Kanälen (a-d) unter Verwendung des Mittelwerts der Intensitätswerte über alle Positionen für die Mehrzahl von Kanälen (a-d) beschrieben werden;
Kombinieren (130) der parametrisierten Verteilungen für die Mehrzahl von Kanälen (a-d), um eine gemeinsame Verteilung für alle der Mehrzahl von Kanälen (a-d) zu erhalten; wobei die gemeinsame Verteilung einen Durchschnitt aller parametrisierten Verteilungen der Mehrzahl von Kanälen darstellt; und
Bestimmen und Anlegen (140) einer Übertragungsfunktion für jeden der Mehrzahl von Kanälen (a-d), so dass die jeweilige Übertragungsfunktion für den jeweiligen Kanal (a-d) die entsprechende Intensitätsverteilung auf die gemeinsame Verteilung abbildet.

2. Das Verfahren (100) gemäß Anspruch 1, bei dem die Parametrisierung (120) und das Bestimmen (140) für vier Kanäle (a-d) durchgeführt wird, die jeweils zu einem bestimmten Nukleotid gehören, und/oder
wobei die Parametrisierung (120) und das Bestimmen (140) für vier Kanäle (a-d) durchgeführt wird, die jeweils gefiltert werden, um einen von vier eindeutigen Fluoreszenzfarbstoffen zu bestimmen, der vier spezifische Nukleotide markiert.

3. Das Verfahren gemäß Anspruch 1 oder 2, bei dem jede Position in der Mehrzahl von Kanälen (a-d) zu ihrer eigenen Sequenz und/oder zu ihrer eigenen DNA-Sequenz gehört.

4. Das Verfahren (100) gemäß einem der vorhergehenden Ansprüche, bei dem der Schritt der Parametrisierung (120) durchgeführt wird unter Verwendung des Teilschritts der Maximale-Wahrscheinlichkeit-Schätzung, Maximale-A-Posteriori-Schätzung, Bestimmen (140) einer Zusammenfassungsstatistik für einen oder mehrere oder alle Kanäle (a-d) oder Bestimmen (140) eines spezifizierten Parametersatzes, der eine Intensitätsverteilung von einem oder mehreren oder allen Kanälen (a-d) beschreibt.

5. Das Verfahren (100) gemäß einem der vorhergehenden Ansprüche, bei dem die jeweilige Übertragungsfunktion eine lineare Transformation der entsprechenden Intensitätsverteilung auf die gemeinsame Verteilung ermöglicht.

6. Das Verfahren (100) gemäß einem der Ansprüche 1 bis 5, bei dem die jeweilige Übertragungsfunktion eine nichtlineare Transformation der entsprechenden Intensitätsverteilung auf die gemeinsame Verteilung ermöglicht.

7. Das Verfahren (100) gemäß einem der vorhergehenden Ansprüche, bei dem die Intensitätsverteilung für einen oder mehrere oder alle der Mehrzahl von Kanälen (a-d) durch eine Gaußsche Verteilung mit einem eindeutigen Mittelwert und einer eindeutigen Standardabweichung beschrieben wird.

8. Das Verfahren (100) gemäß Anspruch 7, bei dem die jeweilige Übertragungsfunktion für den jeweiligen Kanal (11a-11d), beschrieben durch die Gaußsche Verteilung, die mathematischen Operationen zum Subtrahieren des Mittelwerts von der entsprechenden Intensitätsverteilung und/oder Dividieren der entsprechenden Intensitätsverteilung durch die Standardabweichung aufweist.

9. Das Verfahren (100) gemäß Anspruch 6, bei dem die Intensitätsverteilung für einen oder für mehrere oder für alle der Mehrzahl von Kanälen (a-d) durch eine Nicht-Normalverteilung beschrieben ist.

10. Das Verfahren (100) gemäß Anspruch 9, bei dem die jeweilige Übertragungsfunktion eine Protokolltransformation, eine Quadratwurzeltransformation, eine Box-Cox-Transformation oder eine Yeo-Johnson-Transformation ermöglicht.

11. Das Verfahren (100) gemäß einem der vorhergehenden Ansprüche, bei dem die Parametrisierung (120), das Kombinieren (130) und das Bestimmen (140) für jeden einer Mehrzahl von Zyklen individuell durchgeführt wird.

12. Das Verfahren (100) gemäß einem der Ansprüche 1 bis 10, bei dem die Parametrisierung (120), das Kombinieren (130) und das Bestimmen (140) für eine Mehrzahl von Zyklen (11-15) oder alle Zyklen (11-15) durchgeführt wird.

13. Das Verfahren (100) gemäß Anspruch 12, bei dem der Schritt des Bestimmens (140) der Übertragungsfunktion durchgeführt wird, so dass die jeweilige Übertragungsfunktion für den jeweiligen Kanal (a-d) eine Glättungsfunktion aufweist;
wobei die Glättungsfunktion eine maximale Änderung des Intensitätswerts über die Anzahl von Zyklen (11-15) beschreibt und/oder die Glättungsfunktion bestimmt wird (140), so dass der Intensitätswert über die Anzahl von Zyklen (11-15) geglättet wird.

14. Das Verfahren (100) gemäß Anspruch 12 oder 13, bei dem jede jeweilige Übertragungsfunktion eine Normierungsfunktion aufweist;
wobei die Normierungsfunktion bestimmt wird (140), so dass die Intensitätswerte der Mehrzahl von Kanälen (a-d) über die Anzahl von Zyklen (11-15) für alle Kanäle (a-d) normiert werden.

15. Das Verfahren (100) gemäß Anspruch 14, bei dem die jeweilige Normierungsfunktion bestimmt wird (140), falls kein Trend eines oder mehrerer Kanäle (a-d) über die Anzahl von Zyklen (11-15) erfasst wird.

16. Das Verfahren (100) gemäß Anspruch 12, bei dem die gemeinsame Verteilung für alle der Mehrzahl von Kanälen (a-d) durch einen Satz von Funktionen beschrieben wird, die für jeden Kanal eine gemeinsame Verteilungsfunktion aufweisen, wobei zumindest zwei der gemeinsamen Verteilungsfunktionen sich voneinander unterscheiden;
wobei jede jeweilige Übertragungsfunktion bestimmt wird (140), so dass die jeweilige Übertragungsfunktion für den jeweiligen Kanal (a-d) die entsprechende Intensitätsverteilung auf die entsprechende gemeinsame Verteilungsfunktion abbildet.

17. Das Verfahren (100) gemäß Anspruch 15, bei dem die Mehrzahl von gemeinsamen Verteilungsfunktionen bestimmt wird (140), falls ein Trend von einem oder mehreren Kanälen (a-d) über die Anzahl von Zyklen (11-15) erfasst wird.

18. Das Verfahren (100) gemäß Anspruch 16 oder 17, bei dem jede Übertragungsfunktion eine Glättungsfunktion aufweist, wobei die Glättungsfunktion eine maximale Änderung des Intensitätswerts über die Anzahl von Zyklen (11-15) beschreibt und/oder wobei die Glättungsfunktion bestimmt wird (140), so dass der Intensitätswert, der für den jeweiligen Kanal (a-d) verwendet wird, über die Anzahl von Zyklen (11-15) geglättet wird.

19. Das Verfahren (100) gemäß einem der Ansprüche 1 bis 18, bei dem die Intensitätsverteilung über alle Positionen und/oder die parametrisierten Verteilungen die Anzahl von Zählwerten in Korrelation zu jeweiligen Intensitätswerten für den jeweiligen Kanal der Mehrzahl von Kanälen (a-d) beschreiben; und/oder
wobei die gemeinsame Verteilung für zumindest zwei, alle relevanten oder alle Kanäle (a-d) einen Durchschnitt von zumindest zwei, allen relevanten oder allen parametrisierten Verteilungen der Mehrzahl von Kanälen darstellt und/oder wobei die gemeinsame Verteilung für zumindest zwei, alle relevanten oder alle Kanäle eine gemittelte Anzahl von Zählwerten in Korrelation zu einem gemittelten Intensitätswert für zumindest zwei, alle relevanten oder alle Kanäle (a-d) beschreibt.

20. Ein computerlesbares, digitales Speichermedium, auf dem ein Computerprogramm gespeichert ist, mit einem Programmcode, der, wenn derselbe auf einem Computer ausgeführt wird, den Computer veranlasst, das Verfahren gemäß einem der vorhergehenden Ansprüche auszuführen.

21. Vorrichtung zum Durchführen einer Normierung von Intensitätswerten, die erhalten werden, um eine Sequenzierungsanalyse durchzuführen, die folgende Merkmale aufweist:
eine Schnittstelle zum Empfangen einer Mehrzahl von Bilddaten (15a-15d) für eine Mehrzahl von Kanälen (a-d), wobei alle Bilddaten (11a-11d, 15a-15d) für den jeweiligen Kanal (a-d) eine Intensitätsverteilung über alle Positionen des Bildes der Mehrzahl von Bilddaten (11a-11d, 15a-15d) beschreiben;
einen Prozessor, der konfiguriert ist, die Intensitätsverteilung über alle Positionen für die Mehrzahl von Kanälen (a-d) zu parametrisieren, um eine parametrisierte Verteilung für die Mehrzahl von Kanälen (a-d) zu erhalten, und konfiguriert ist, die parametrisierte Verteilung für die Mehrzahl von Kanälen (a-d) zu kombinieren, um eine gemeinsame Verteilung für alle der Mehrzahl von Kanälen (a-d) zu erhalten; wobei die parametrisierten Verteilungen für die Mehrzahl von Kanälen (a-d) unter Verwendung des Mittelwerts der Intensitätswerte über alle Positionen für die Mehrzahl von Kanälen (a-d) beschrieben werden; wobei die gemeinsame Verteilung einen Durchschnitt aller parametrisierten Verteilungen der Mehrzahl von Kanälen darstellt; und
wobei der Prozessor konfiguriert ist, für jeden der Mehrzahl von Kanälen (a-d) eine Übertragungsfunktion zu bestimmen und anzulegen (140), so dass die jeweilige Übertragungsfunktion für den jeweiligen Kanal (a-d) die entsprechende Intensitätsverteilung auf die gemeinsame Verteilung abbildet.

## Revendications

1. Procédé mis en oeuvre par ordinateur (100) pour effectuer une normalisation des valeurs d'intensité obtenues pour effectuer une analyse de séquençage, le procédé (100) comprend les étapes consistant à:
recevoir (110) une pluralité de données d'image (11a à 11d, 15a à 15d) pour une pluralité de canaux (a à d), chaque donnée d'image (11a à 11d, 15a à 15d) décrivant, pour le canal (a à d) respectif, une distribution d'intensité sur toutes les positions d'une image de la pluralité de données d'image (11a à 11d, 15a à 15d);
paramétrer (120) la distribution d'intensité sur toutes les positions pour la pluralité de canaux (a à d) pour obtenir des distributions paramétrées pour la pluralité de canaux (a à d); où les distributions paramétrées pour la pluralité de canaux (a à d) sont décrites à l'aide de la moyenne des valeurs d'intensité sur toutes les positions pour la pluralité de canaux (a à d);
combiner (130) les distributions paramétrées pour la pluralité de canaux (a à d) pour obtenir une distribution commune pour l'ensemble de la pluralité de canaux (a à d); où la distribution commune représente une moyenne de toutes les distributions paramétrées de la pluralité de canaux; et
déterminer et appliquer (140), pour chacun de la pluralité de canaux (a à d), une fonction de transfert telle que la fonction de transfert respective pour le canal (a à d) respectif mappe la distribution d'intensité correspondante sur la distribution commune.

2. Procédé (100) selon la revendication 1, dans lequel la paramétrisation (120) et la détermination (140) sont effectuées pour quatre canaux (a à d) appartenant, chacun, à un nucléotide spécifique, et/ou
dans lequel la paramétrisation (120) et la détermination (140) sont effectuées pour quatre canaux (a à d) filtrés, chacun, pour déterminer l'un de quatre colorants fluorescents distincts marquant quatre nucléotides spécifiques.

3. Procédé selon la revendication 1 ou 2, dans lequel chaque position dans la pluralité de canaux (a à d) appartient à sa propre séquence et/ou à sa propre séquence d'ADN.

4. Procédé (100) selon l'une des revendications précédentes, dans lequel l'étape de paramétrisation (120) est effectuée à l'aide de la sous-étape consistant à estimer la vraisemblance maximale, estimer l'a posteriori maximum, déterminer (140) une statistique sommaire pour un ou plusieurs ou tous les canaux (a à d) ou déterminer (140) un ensemble de paramètres spécifié décrivant une distribution d'intensité d'un ou plusieurs ou de tous les canaux (a à d).

5. Procédé (100) selon l'une des revendications précédentes, dans lequel la fonction de transfert respective permet une transformation linéaire de la distribution d'intensité correspondante en la distribution commune.

6. Procédé (100) selon l'une des revendications 1 à 5, dans lequel la fonction de transfert respective permet une transformation non linéaire de la distribution d'intensité correspondante en la distribution commune.

7. Procédé (100) selon l'une des revendications précédentes, dans lequel la distribution d'intensité pour un ou plusieurs ou l'ensemble de la pluralité de canaux (a à d) est décrite par une distribution gaussienne avec une moyenne distincte et un écart standard distinct.

8. Procédé (100) selon la revendication 7, dans lequel la fonction de transfert respective pour le canal (11a à 11d) respectif décrit par la distribution gaussienne comprend les opérations mathématiques consistant à soustraire la moyenne de la distribution d'intensité correspondante et/ou à diviser la distribution d'intensité correspondante par l'écart standard.

9. Procédé (100) selon la revendication 6, dans lequel la distribution d'intensité pour un ou pour plusieurs ou pour l'ensemble de la pluralité de canaux (a à d) est décrite par une distribution non normale.

10. Procédé (100) selon la revendication 9, dans lequel la fonction de transfert respective permet une transformation logarithmique, une transformation de racine carrée, une transformation de Box-Cox ou une transformation de Yeo-Johnson.

11. Procédé (100) selon l'une des revendications précédentes, dans lequel la paramétrisation (120), la combinaison (130) et la détermination (140) sont effectuées individuellement pour chacun d'une pluralité de cycles.

12. Procédé (100) selon l'une des revendications 1 à 10, dans lequel la paramétrisation (120), la combinaison (130) et la détermination (140) sont effectuées pour une pluralité de cycles (11 à 15) ou pour tous les cycles (11 à 15).

13. Procédé (100) selon la revendication 12, dans lequel l'étape de détermination (140) de la fonction de transfert est effectuée de sorte que la fonction de transfert respective pour le canal (a à d) respectif comprenne une fonction de lissage;
dans lequel la fonction de lissage décrit une variation maximale de la valeur d'intensité sur le nombre de cycles (11 à 15) et/ou la fonction de lissage est déterminée (140) de sorte que la valeur d'intensité soit lissée sur le nombre de cycles (11 à 15).

14. Procédé (100) selon la revendication 12 ou 13, dans lequel chaque fonction de transfert respective comprend une fonction de normalisation;
dans lequel la fonction de normalisation est déterminée (140) de sorte que les valeurs d'intensité de la pluralité de canaux (a à d) soient normalisées sur le nombre de cycles (11 à 15) pour tous les canaux (a à d).

15. Procédé (100) selon la revendication 14, dans lequel la fonction de normalisation respective est déterminée (140) s'il n'est pas détecté de tendance d'un ou plusieurs canaux (a à d) sur le nombre de cycles (11 à 15).

16. Procédé (100) selon la revendication 12, dans lequel la distribution commune pour l'ensemble de la pluralité de canaux (a à d) est décrite par un ensemble de fonctions comprenant, pour chaque canal, une fonction de distribution commune, dans lequel au moins deux des fonctions de distribution commune diffèrent l'une de l'autre;
dans lequel chaque fonction de transfert respective est déterminée (140) de sorte que la fonction de transfert respective pour le canal (a à d) respectif mappe la distribution d'intensité correspondante sur la fonction de distribution commune correspondante.

17. Procédé (100) selon la revendication 15, dans lequel la pluralité de fonctions de distribution communes est déterminée (140) s'il est détecté une tendance d'un ou plusieurs canaux (a à d) sur le nombre de cycles (11 à 15).

18. Procédé (100) selon la revendication 16 ou 17, dans lequel chaque fonction de transfert comprend une fonction de lissage, dans lequel la fonction de lissage décrit une variation maximale de la valeur d'intensité sur le nombre de cycles (11 à 15) et/ou dans lequel la fonction de lissage est déterminée (140) de sorte que la valeur d'intensité utilisée pour le canal (a à d) respectif soit lissée sur le nombre de cycles (11 à 15).

19. Procédé (100) selon l'une des revendications 1 à 18, dans lequel la distribution d'intensité sur toutes les positions et/ou les distributions paramétrées décrivent le nombre de comptages en corrélation avec les valeurs d'intensité respectives pour le canal respectif de la pluralité de canaux (a à d); et/ou
dans lequel la distribution commune pour au moins deux, tous les canaux pertinents ou tous les canaux (a à d) représente une moyenne d'au moins deux, toutes les distributions pertinentes ou toutes les distributions paramétrées de la pluralité de canaux et/ou dans lequel la distribution commune pour au moins deux, tous les canaux pertinents ou tous les canaux décrit un nombre moyen de comptages en corrélation avec les valeurs d'intensité moyennes pour au moins deux, tous les canaux pertinents ou tous les canaux (a à d).

20. Support de mémoire numérique lisible par ordinateur présentant, y mémorisé, un programme d'ordinateur présentant un code de programme qui, lorsqu'il est exécuté sur un ordinateur, amène l'ordinateur à réaliser le procédé selon l'une des revendications précédentes.

21. Appareil pour effectuer une normalisation des valeurs d'intensité obtenues pour effectuer une analyse de séquençage, comprenant:
une interface destinée à recevoir une pluralité de données d'image (15a à 15d) pour une pluralité de canaux (a à d), chaque donnée d'image (11a à 11d, 15a à 15d) décrivant, pour le canal (a à d) respectif, une distribution d'intensité sur toutes les positions de l'image de la pluralité de données d'image (11a à 11d, 15a à 15d);
un processeur configuré pour paramétrer la distribution d'intensité sur toutes les positions pour la pluralité de canaux (a à d) pour obtenir une distribution paramétrée pour la pluralité de canaux (a à d) et configuré pour combiner la distribution paramétrée pour la pluralité de canaux (a à d) pour obtenir une distribution commune pour l'ensemble de la pluralité de canaux (a à d); où les distributions paramétrées pour la pluralité de canaux (a à d) sont décrites à l'aide de la moyenne des valeurs d'intensité sur toutes les positions pour la pluralité de canaux (a à d); où la distribution commune représente une moyenne de toutes les distributions paramétrées de la pluralité de canaux; et
dans lequel le processeur est configuré pour déterminer et appliquer (140), pour chacun de la pluralité de canaux (a à d), une fonction de transfert de sorte que la fonction de transfert respective pour le canal (a à d) respectif mappe la distribution d'intensité correspondante sur la distribution commune.
